# EUROPEAN PATENT APPLICATION

(11) **EP 3 711 478 A1**
(43) Date of publication of application: **23.09.2020**
(21) Application number: 19164363.4
(22) Date of filing: 21.03.2019
(51) Int. Cl.: A01H 1/04, G16B 40/00, G16B 5/00, G01N 33/50

(54) **METHOD FOR PREDICTING YIELD LOSS OF A CROP PLANT**

(71) Applicant: BASF SE, 67056 Ludwigshafen am Rhein (DE)
(72) Inventor: HEILLMANN, Monika, 67117 Limburgerhof (DE); PUENTE, Pilar, 67117 Limburgerhof (DE); THIMM, Oliver, 67056 Ludwigshafen (DE); PROCTOR, Iain, 67117 Limburgerhof (DE); SRINIVAS, Girish, 67117 Limburgerhof (DE)

(57) **Abstract**

Method for predicting yield loss of a crop plant, comprising the steps:
receiving metabolite measurements (M) of the crop plant (50);
determining new metabolite features (Mn) combining the received metabolite measurements (M);
determining a model (13) by a machine learning algorithm based on the new metabolite features (Mn); and
determining yield loss prediction data (Yp) of the crop plant (50) using the determined model (13).

## Description

### FIELD OF INVENTION

The present invention relates to a method for predicting yield loss of a crop plant, a control unit, a yield evaluation platform, a plant breeding method, a farming method and a use of new metabolite features.

### BACKGROUND OF THE INVENTION

The world is faced with rapid population growth and expected to reach 9.6 billion by 2050. To meet the growing demand for food, we need to secure our food supply by producing crops with more yield on the same amount of farmland. In addition to protecting crops against pests and disease, growers must also optimize yields and maintain plant health by managing environmental stress while meeting evolving societal expectations of agriculture. Enabling this full yield potential requires solutions beyond conventional crop protection.

Abiotic stress for the crop plant due to environmental factors is almost always not preventable during cultivation of the crop plant. However, abiotic stress during the growth of the crop plant, usually leads to loss in yield of the crop plant. Therefore, different variants of crop plants are researched, which comprise an improved resistance to abiotic stress.

In research variables measured such as biomass are not yield predicting and thus not sufficient for enabling decisions.

The chemical and/or biological search space is too large for a high throughput screening in the crop plant of interest for yield or stress tolerance under controlled conditions. Therefore, it is difficult to predict the expected yield of a crop plant candidate.

### SUMMARY OF THE INVENTION

Thus, there is a need for an improved method for predicting yield loss of a crop plant.

The object of the present invention is solved with the subject matter of the independent claims, wherein further embodiments are incorporated in the dependent claims. It should be noted that the following described aspects and examples of the invention apply also for the method for predicting yield loss of a crop plant, the control unit, the yield evaluation platform, the plant breeding method and the use of new metabolite features.

An aspect relates to a method for predicting yield loss of a crop plant, comprising the steps:
receiving metabolite measurements of the crop plant;
determining new metabolite features combining the received metabolite measurements;
determining a model by a machine learning algorithm based on the new metabolite features; and
determining yield loss prediction data of the crop plant using the determined model.

The term "crop plant", as used herein, comprises a plant to be cultivated in a crop field. Different variations of crop plants to be analyzed and/or used for further breeding are also described as "crop plant candidates".

The term "model", as used herein, comprises a mathematical model representing a plant, in particular a crop plant.

The term "crop cycle", as used herein, comprises the growth cycle of a crop plant including a vegetative growth stage and a reproductive growth stage.

The term "biomarker", as used herein, comprises a measurable indicator of a biological state or condition, in particular of a crop plant. While a measured metabolite feature itself does not automatically qualify as a biomarker, the determined new metabolite feature does qualify as a biomarker.

The model preferably is a machine learning model being trained on a training dataset containing the measured as well as new metabolite features. Test datasets and validation datasets are used to predict the model performance. The machine learning model predicts whether a crop plant will have a yield loss or not.

The machine learning algorithm preferably comprises decision trees, naive bayes classifications, nearest neighbors, neural networks, convolutional neural networks, generative adversarial networks, support vector machines, linear regression, logistic regression, random forest and/or gradient boosting algorithms.

The machine learning algorithm preferably is carried out by a so called artificial intelligence module. The artificial intelligence module is an entity that processes one or more inputs into one or more outputs by means of an internal processing chain that typically has a set of free parameters. The internal processing chain may be organized in interconnected layers that are traversed consecutively when proceeding from the input to the output.

Many artificial intelligence modules are organized to process an input having a high dimensionality into an output of a much lower dimensionality. Such a module is termed "intelligent" because it is capable of being "trained." The module may be trained using records of training data. A record of training data comprises training input data and corresponding training output data. The training output data of a record of training data is the result that is expected to be produced by the module when being given the training input data of the same record of training data as input. The deviation between this expected result and the actual result produced by the module is observed and rated by means of a "loss function". This loss function is used as a feedback for adjusting the parameters of the internal processing chain of the module. For example, the parameters may be adjusted with the optimization goal of minimizing the values of the loss function that result when all training input data is fed into the module and the outcome is compared with the corresponding training output data. The result of this training is that given a relatively small number of records of training data as "ground truth", the module is enabled to perform its job well for a number of records of input data that higher by many orders of magnitude.

The metabolite measurements are combined to new metabolite features. However, the process of combining the metabolite measurements is preferably achieved by combining the metabolite measurements to a relatively general class and separate those classes again to more specific classes, thereby obtaining the new metabolite feature.

The model is preferably trained and tested on measured variables coming from the reproductive stage as well as vegetative stage of the crop plant. Preferably, at least one crop cycle, in particular one year, is excluded from the training of the model in order to test the model. Preferably, the model is trained and tested to predict two classes of yield: "yield loss" or "no yield loss".

In a preferred embodiment, the model determines the yield loss prediction data, preferably data from the vegetative stage, for the next crop cycle, in particular the next year. This step is repeated each time for training and testing the vegetative data of the excluded year to understand the model performance.

In a preferred embodiment, the accuracy of the model is bigger than 75%, further preferably in range of 79% to 86%.

Thus, the search space for identifying the most relevant biomarkers, in particular new metabolite features, can be reduced. Therefore, the yield loss prediction of the crop plant can be improved.

Thus, the plant performance is improved by improving precision and efficiency in plant breeding, crop trait development for crops as well as development of biostimulants. It further helps farming practice in farmers' decisions such as irrigation.

Preferably, the described method comprises the steps:
receiving hyperspectral data of the crop plant;
determining vegetation indices, relating to a combination of spectral bands from the crop plant, preferably having physiological meaning, from the hyperspectral data; and
determining a model by a machine learning algorithm based on the new metabolite features and/or the vegetation indices.

In a preferred embodiment, in addition to the hyperspectral data of the crop plant, thermography data of the crop plant as well as data about crop plant volume and/or plant height are received and used by the machine learning algorithm to determine the model.

In a preferred embodiment the hyperspectral data is gathered by spectral imaging with visible and near-infrared (VNIR) and/or short wavelength infrared (SWIR), the thermography data is gathered by thermography imaging.

Preferably, the hyperspectral data is provided by a hyperspectral sensor, like a type of camera, that flies over the crop field in an aeroplane or a drone. The hyperspectral sensor preferably produces images where every pixel has full spectral information and the data are retrieved in spectral bands, also called wavelengths. The hyperspectral data preferably is a 3D data set, where first and second dimensions are the surface of the object and on top every layer is the information in one spectral band at a time. Once the wavelength is acquired, it is used to calculate vegetation indices.

Thus, the prediction of the yield loss of the crop plant can be further improved.

Preferably, the described method comprises the steps:
determining the metabolite measurements based on a crop plant sample by chromatography, preferably polar gas chromatography, lipid gas chromatography, polar liquid chromatography and/or lipid liquid chromatography.

Thus, the prediction of the yield loss of the crop plant can be further improved.

Preferably, the hyperspectral data and/or metabolite measurements are based on crop field trials with different levels of abiotic stress, preferably drought stress.

The term "crop field trial", as used herein, comprises the cultivation of different variations of crop plants of the same crop plant type under predetermined conditions in order to evaluate the performance of the different variations of crop plants, in particular for breeding purposes, trait selection purposes and/or biostimulant treatment selection purposes.

In order to develop a method for predicting yield loss of a plant, a common parameter has to be found that is measurable at both stages of plant growth, the vegetative stage and the reproductive stage, in the crop field.

Preferably, biomarkers, in particular new metabolite features, are identified to build a link between vegetative and reproductive stages within the crop plant in the field that account for the abiotic stress, in particular the drought stress, and mainly yield impact. To find these potential biomarkers based on crop field trials, preferably at least two simultaneous crop field trials are set up using a randomized block design with at least three different levels of abiotic stress, preferably with a level of no abiotic stress as control. Preferably, the plants are subjected to these different treatment levels at vegetative or reproductive growth stages respectively.

The crop field trials are preferably conducted over at least two years, further preferably at least two crop cycles. Preferably, within one crop cycle, crop plant samples of the crop field trials of several, preferably at least three, time points are analyzed. In a preferred embodiment, the crop field trials comprise at least two different varieties of plant seeds, preferably corn seeds. Preferably, at least one common variety of plant seeds is used throughout the crop field trials. In other words, one common variety of plant seeds is used over several, in particular all, crop cycles. At the end of the crop cycle, the actual yield per plant in the plant crop field stressed at both stages of plant growth, the vegetative growth stage and the reproductive growth stage, is measured.

Thus, the prediction of the yield loss of the crop plant can be further improved.

In a preferred embodiment, the crop field trials are performed during a vegetative and/or a reproductive growth stage of the plant.

Preferably, the described method comprises the steps:
determining the new metabolite features combining the received metabolite measurements based on assignments of the received metabolite measurements to different ontologies and/or based on a ratio between product metabolites and substrate metabolites.

Preferably, the ontologies comprise different levels, wherein preferably different levels are associated to different generalizations of metabolite measurements.

Therefore, the metabolite measurements can be further grouped.

Thus, the prediction of the yield loss of the crop plant can thus be further improved.

In a preferred embodiment, a first level of ontologies preferably comprises one of amino acids and related or organic acids or carbohydrates and related or complex lipids, fatty acids and related or secondary metabolism or phytohormones or nucleobases and related or miscellaneous. A second level of ontologies as an example of a subgroup of carbohydrates and related comprises sugar alcohol or free sugars or sugar phosphates or sugar acids.

Preferably, the described method comprises the steps:
validating the yield loss prediction data and providing validation data by comparing the yield loss prediction data with the actual yield data of the respective crop plant; and
adjusting the model based on the validation data.

Thus, the prediction of the yield loss of the crop plant can be further improved.

Preferably, the described method comprises the steps:
adjusting a parametrization of a machine learning algorithm determining the model based on the validation data;
wherein the machine learning algorithm comprises several combined machine learning algorithms, in particular three combined machine learning algorithms.

Thus, the prediction of the yield loss of the crop plant can be further improved.

Preferably, the described method comprises the steps:
determining a best metabolite feature from the metabolite feature based on the validation data; wherein
the best metabolite feature comprises the metabolite measurements with the highest impact on the expected yield loss; and
wherein preferably the best metabolite feature comprises metabolite measurements extracted by polar gas chromatography.

In a preferred embodiment, the machine learning algorithm is only trained by metabolite measurements, which already have been proven to be especially relevant to qualify as a good predicting biomarker.

In a preferred embodiment, the yield loss prediction data preferably comprises the states "yield loss" and "no yield loss".

Thus, the prediction of the yield loss of the crop plant can be further improved.

Preferably, the yield loss prediction data is determined within the vegetative growth stage of the plant.

Thus, a potential yield loss due to abiotic stress of the analyzed variation of crop plant can already be predicted within the vegetative growth stage. Therefore, a lot of time and expenses can be saved, especially since the costs for cultivating the crop plant increases by time. Crop plants with high predicted yield loss due to abiotic stress therefore can be sorted out earlier.

A further aspect relates to a control unit being configured for executing a method, described herein.

The control unit may refer to a data processing element such as a microprocessor, microcontroller, crop field programmable gate array (FPGA), central processing unit (CPU), digital signal processor (DSP) capable of receiving crop field data, e.g. via a universal service bus (USB), a physical cable, Bluetooth, or another form of data connection.

A further aspect relates to a yield evaluation platform, comprising a hyperspectral sensor configured for determining hyperspectral data of a plant, a profiling platform configured for determining metabolite measurements from a crop plant sample and a control unit, as described herein.

A further aspect relates to a plant breeding method, comprising the steps:
cultivating crop plants;
determining yield loss prediction data of the respective crop plant using the method for predicting yield loss, as described herein; and
selecting the crop plants with a predicted yield loss below a predetermined threshold for future breeding.

In a preferred embodiment, the plant breeding method is not limited to crop plants, but any kind of germplasm.

Therefore, a breeder can select the crop plants with the highest predicted yield potential for further breeding in an improved way.

In a preferred embodiment, a trait selecting method is provided, comprising the steps:
cultivating crop plants;
determining yield loss prediction data of the respective crop plant using the method for predicting yield loss, as described herein; and
selecting the trait with a predicted yield loss for the crop plant below a predetermined threshold.

Therefore, the trait with the highest predicted yield potential for the crop plant can be selected in an improved way.

In a preferred embodiment, a biostimulant treatment selecting method is provided, comprising the steps:
cultivating crop plants;
determining yield loss prediction data of the respective crop plant using the method for predicting yield loss, as described herein; and
selecting the biostimulant treatment with a predicted yield loss for the crop plant below a predetermined threshold.

The term "biostimulants" comprises substances and/or microorganisms applied to crop plants that stimulate natural processes in the plant to enhance nutrient uptake or nutrient efficiency, improve tolerance to abiotic stress or crop quality. In agriculture, biostimulation is complementary to crop plant nutrition, for example using fertilizers and crop protection, being products to control pathogens, pests and/or weeds.

Therefore, the biostimulant treatment with the highest predicted yield potential for the crop plant can be selected in an improved way.

A further aspect relates to a farming method, comprising the steps:
determining yield loss prediction data of the respective crop plant using the method for predicting yield loss, as described herein;
providing farmers with an expected yield loss of the crop plant depending on the determined yield loss prediction data; and
adjusting the farming conditions by the farmer depending on the expected yield loss of the crop plant.

Therefore, a farmer can adjust the farming conditions of the respective field, in order to prevent unwanted yield loss.

Preferably, the farmer cultivates the crop plant for a certain amount of time, for example 15 days. After that, the farmer provides a sample of the crop plant to an external service, for example a vendor. The external service executes the method for yield loss prediction, as described herein, preferably by a yield evaluation platform, as described herein, on the provided sample of the crop plant. The external service then provides the farmer with a predicted yield loss based on yield loss prediction data determined by the method for yield loss prediction. The farmer then adjusts the farming condition on his field depending on the predicted yield loss, for example by increasing the watering amount and/or using yield enhancing products.

A further aspect relates to a use of new metabolite features combining received metabolite measurements of a crop plant sample for yield loss prediction of the crop plant.

Preferably, the use of new metabolite features combining received metabolites of a crop plant sample for yield loss prediction of the plant is based on assignments of the received metabolite measurements to different ontologies and/or based on a ratio between product metabolite measurements and substrate metabolite measurements.

In a preferred embodiment, a computer program is preferably provided that when it is executed on a control unit, as described herein, instructs the control unit to execute steps of a method, as described herein.

In a preferred embodiment, a computer readable storage medium is preferably provided, being configured to store a computer program, as described herein.

In a preferred embodiment, a crop field management system is provided, being configured for detection of a crop failure. Preferably, the crop field management system is provided with yield loss prediction data by the control unit and is configured for determining the crop failure if the predicted yield loss does exceed a predetermined crop failure threshold.

The crop field management system is further preferably configured for taking actions counteracting the predicted crop failure, if the crop field management system determines the crop failure. Preferably the actions counteracting the prediction crop failure comprises reducing abiotic stress factors like drought, extreme temperature, UV-radiation and/or nutrient deficiency, and/or applying crop protection products like fungicides, herbicides and/or insecticides and/or applying yield enhancing chemicals, microbes, natural compounds and/or natural extracts.

Thus, the yield of plants with expected low yield/high yield loss, can be improved.

Alternatively, the crop field management system is further preferably configured for removing plant candidates, if the crop field management system determines crop failure. Removing crop plant candidates with predicted high yield loss helps reducing the maintenance costs of the crop field.

In a preferred embodiment, a plant breeding management system is provided, being configured for determining yield loss prediction data of the respective crop plants using the method for predicting yield loss, as described herein. The plant breeding management system is further configured for selecting the crop plants with a predicted yield loss below a predetermined threshold for future breeding cycles.

In a preferred embodiment, the plant breeding management system is not limited to crop plants, but any kind of germplasm.

Preferably, the plant breeding management system is configured for controlling a plant treatment device, which is configured for treating plants. The plant treatment device comprises means for sucking, pulling and/or stamping plants. Preferably, the plant treatment device is mounted on an automated unmanned working machine like a drone.

In a preferred embodiment, the crop field management system is configured for cultivating crop plants, determining yield loss prediction data of the respective crop plant using the method for predicting yield loss, as described herein and selecting the trait with a predicted yield loss for the crop plant below a predetermined threshold.

In a preferred embodiment, the crop field management system is configured cultivating crop plants, determining yield loss prediction data of the respective crop plant using the method for predicting yield loss, as described herein and selecting the biostimulant treatment with a predicted yield loss for the crop plant below a predetermined threshold.

Advantageously, the benefits provided by any of the above aspects equally apply to all of the other aspects and vice versa. The above aspects and examples will become apparent from and be elucidated with reference to the embodiments described hereinafter.

### BRIEF DESCRIPTION OF THE DRAWINGS

Exemplary embodiments will be described in the following with reference to the following drawings:
- Fig. 1: shows a schematic diagram of a yield evaluation platform;
- Fig. 2: shows a schematic diagram of a metabolite profiling process;
- Fig. 3: shows a schematic diagram of the combination of metabolite measurements;
- Fig. 4: shows a schematic diagram of a control unit; and
- Fig. 5: shows a schematic diagram of a method for predicting yield loss of a crop plant.

### DETAILED DESCRIPTION OF EMBODIMENTS

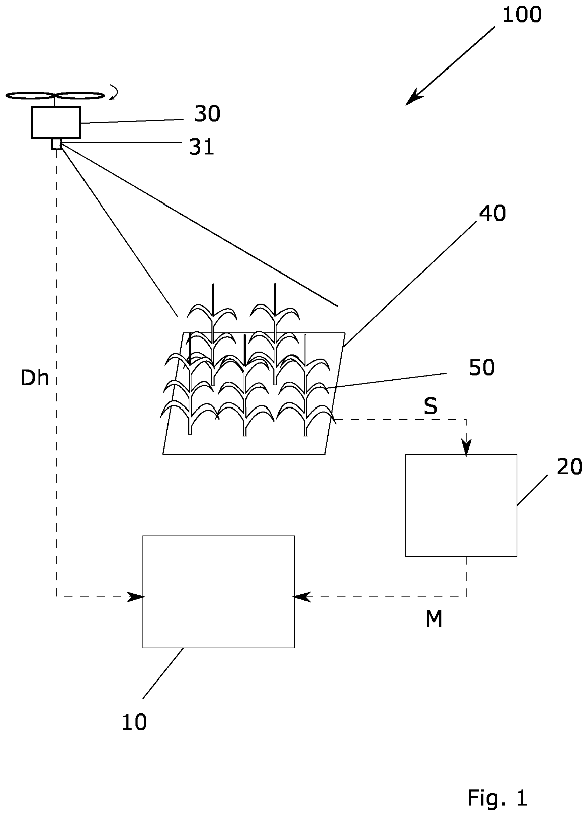

Fig.1 shows a yield evaluation platform 100 comprising a crop field 40 cultivating crop plants 50. In this example, the crop plants 50 comprise corn plants. In order to make assumptions regarding the expected yield of the crop plants 50, measurable indicators, so called biomarkers, have to be found.

Developing relevant biomarker, different crop field trials have to be run on different setups, in particular on different levels of abiotic stress like drought stress. Therefore, different crop plants 50 or areas of crop plants 50 on the crop field 40 are continuously stressed by constant levels of drought stress. For example two simultaneous field trials are set up using randomized block design with three different levels of water treatment. As a comparison, a control group of crop plants 50, which are not stressed is also added. Crop plants 50 are subjected to these different treatment levels at vegetative or reproductive growth stages, respectively. The same trials are conducted for several subsequent years. The crop plant trails are run from the vegetative growth stage, about thirty days, through the reproductive phase, about sixty days, of the crop plant 50. Ideally, a biomarker can be found that has a high field predictive power and allows assumptions on the yield or the yield loss of a crop plant 50 within the vegetative growth stage.

The crop field trials use drought stress as abiotic stress to evaluate different biomarkers on their predictive power regarding the yield loss of the crop plant 50 relating to the drought stress. The expected yield loss of a crop plant 50 increases with the amount of drought stress applied to the crop plant 50.

For each experimental setup in crop field trials, at least two different varieties of crop seeds are used, but at least one common variety is maintained throughout the crop field trials.

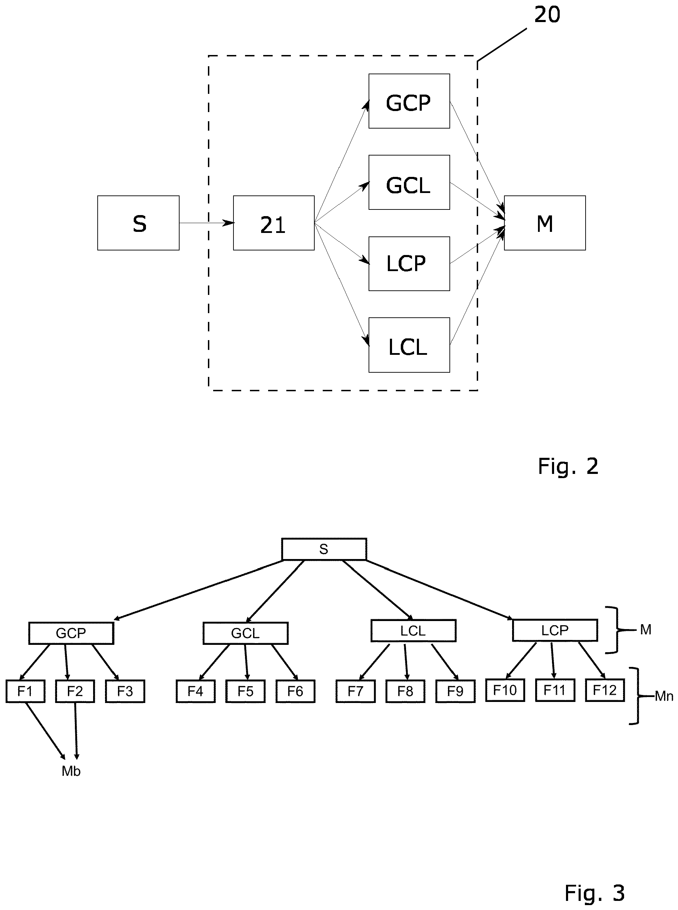

For finding a valid biomarker, on different stages of the crop field trial, crop plant samples S of the different crop plants 50 are taken. For example, the crop plant sample S is corn leaf tissue. For example, crop plant samples S from three different time points are taken. The crop plant samples S are then provided to a profiling platform 20, generating metabolite measurements M from the crop plant samples S and providing them to the control unit 10, as described in detail in Fig. 2.

Additional information about the crop field 40 is gathered by remote sensing. Therefore, a hyperspectral sensor 31, which is preferably mounted on a drone 30, gathers hyperspectral and thermal information, as well as information about the volume and the height of the crop plants 50. The hyperspectral sensor 31 is therefore configured for gathering hyperspectral data Dh, in particular by spectral imaging with visible and near-infrared (VNIR) and/or short wavelength infrared (SWIR). The hyperspectral data Dh, is provided to the control unit 10.

Instead of a drone 30, the hyperspectral sensor 31 can be mounted on any manned or unmanned working machine.

Fig. 2 shows a schematic diagram of a metabolite profiling process using the profiling platform 20. A crop plant sample S is provided to the profiling platform 20. The profiling platform 20 comprises a preparation unit 21, being configured for freeze-drying and/or milling the crop plant sample S and being configured for extraction and separation of the crop plant sample S in lipid and polar phase. One extraction of the crop plant sample S thereby delivers the whole spectrum of metabolite measurements M.

As is shown, from a single crop plant sample S four different data sets can be received. The total number of metabolite measurements M identified with all four data sets is around 750 metabolite measurements M. The metabolite measurements M are determined by polar gas chromatography GCP, lipid gas chromatography GCL, polar liquid chromatography LCP and/or lipid liquid chromatography LCL and then provided to the control unit 10.

Fig. 3 shows a schematic diagram of the combination of metabolite measurements M. The metabolite measurements M are assigned to different ontologies, by which metabolite measurements M are combined.

In this example, the metabolite features Mn determined by polar gas chromatography GCP are partly assigned to a first ontology F1, for example comprising organic acids, amino acids and related and carbohydrates and related.

In this example, the metabolite features Mn determined by polar gas chromatography GCP are partly assigned to a second ontology F2, for example comprising sugar alcohols, sugar phosphates and free sugars.

In this example, the metabolite features Mn determined by polar gas chromatography GCP are defined by different ratios between two metabolite measurements M that is defined as product and substrate in an enzyme mapping F3.

Using this method, new metabolite features Mn are determined combining the received metabolite measurements M. The new metabolite features Mn are then provided to a model 13 of the control unit 10.

Fig. 3 shows that similar assignments are done for lipid gas chromatography GCL, polar liquid chromatography (LCP) and lipid liquid chromatography (LCL) determining fourth to twelfth ontologies F4 to F12.

The different ontologies F1 to F12 are validated and best new metabolites features Mb are determined from the new metabolite features Mn, wherein the best metabolite features Mb comprises the metabolite features Mn with the highest impact on the expected yield loss. In this case the first ontology F1 and the second ontology F2 are the best new metabolite features Mb.

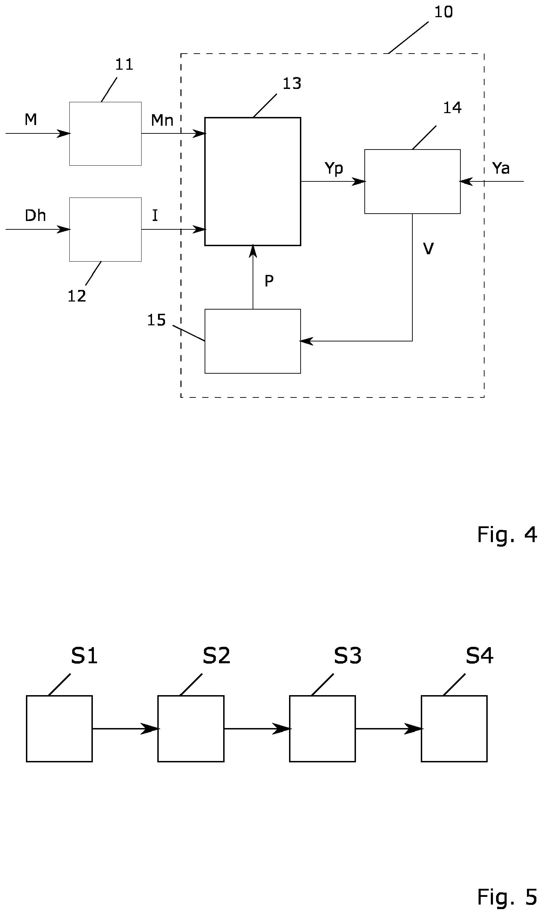

Fig. 4 shows a schematic diagram of the control unit 10, comprising the ontology unit 11, vegetation indices unit 12, the model 13, a yield prediction unit 14 and a machine learning unit 15. As described above, the new metabolite features Mn are provided to the model 13. Additionally, the hyperspectral data Dh are also provided to the vegetation indices unit 12. The vegetation indices unit 12 calculates vegetation indices I. The vegetation indices I are knowledge driven variables as they have physiological meaning in crop plants. The vegetation indices I are then provided to the model 13.

The model 13 is provided with parameters P from the machine learning unit 14. Based on the parameters P and the provided data from the ontology unit 11 and the hyperspectral sensor 31, the model 13 is trained. The model 13 then is used to provide a yield prediction data Yp of the respective crop plant 50. The model is preferably trained and tested to predict two classes of yield: "yield loss" and "no yield loss". The yield prediction data Yp of the model 13 is provided to the validation unit 14. If available, the validation unit 14 additionally is provided with actual yield data Ya. The validation unit 14 then compares the yield prediction data Yp with the actual yield data Ya and determines validation data V, representing the accuracy of the yield prediction data Yp. The validation data V is provided to the machine learning unit 15, which adjusts the parameters P provided to the model 13 based on the validation data V.

The control unit 10, the ontology unit 11, the vegetation indices unit 12, the model 13, the validation unit 14 and/or the machine learning unit 15 may refer to a data processing element such as a microprocessor, microcontroller, crop field programmable gate array (FPGA), central processing unit (CPU), digital signal processor (DSP) capable of receiving crop field data, e.g. via a universal service bus (USB), a physical cable, Bluetooth, or another form of data connection. The respective units may be several independent devices. However, more or all respective units may be integrated into one device.

Fig. 5 shows a schematic diagram of a method for predicting yield loss of a crop plant 50.

In step S1, metabolite measurements M of the crop plant 50 are received. In step S2, new metabolite features Mn are determined combining the received metabolite measurements M. In step S3, a model 13 is determined by a machine learning algorithm based the new metabolite features Mn. In step S4, yield loss prediction data Yp of the crop plant 50 is determined using the determined model 13.

### REFERENCE SIGNS

- 10: control unit
- 11: ontology unit
- 12: vegetation indices unit
- 13: model
- 14: validation unit
- 15: machine learning unit
- 20: profiling platform
- 21: preparation unit
- 30: drone
- 31: hyperspectral sensor
- 40: crop field
- 50: crop plant
- 100: yield evaluation platform
- S: crop plant sample
- Yp: yield prediction data
- Ya: actual yield data
- P: parameter
- V: validation data
- M: metabolite feature
- Mn: new metabolite features
- Mb: best metabolite features
- GCP: polar gas chromatography
- GCL: lipid gas chromatography
- LCP: polar liquid chromatography
- LCL: lipid liquid chromatography
- F1: first ontology
- F2: second ontology
- F3: ratio between product metabolites and substrate metabolites
- F4 to F12: Fourth to twelfth ontology
- Dh: hyperspectral data
- I: indices
- S1: receiving metabolite measurements
- S2: determining new metabolite features
- S3: determining a model
- S4: determining yield loss prediction data

## Claims

1. Method for predicting yield loss of a crop plant, comprising the steps:
receiving (S1) metabolite measurements (M) of the crop plant (50);
determining (S2) new metabolite features (Mn) combining the received metabolite measurements (M);
determining (S3) a model (13) by a machine learning algorithm based on the new metabolite features (Mn); and
determining (S4) yield loss prediction data (Yp) of the crop plant (50) using the determined model (13).

2. Method of claim 1, comprising the steps:
receiving hyperspectral data (Dh) of the crop plant (50);
determining vegetation indices (I), relating to a combination of spectral bands from the crop plant (50), preferably having physiological meaning, from the hyperspectral data (Dh); and
determining a model (13) by a machine learning algorithm based on the new metabolite features (Mn) and/or the vegetation indices (I).

3. Method of any of the preceding claims, comprising the step:
determining the metabolite measurements (M) based on a crop plant sample (S) by chromatography, preferably polar gas chromatography (GCP), lipid gas chromatography (GCL), polar liquid chromatography (LCP) and/or lipid liquid chromatography (LCL).

4. Method of any of the preceding claims, wherein
the hyperspectral data (Dh) and/or metabolite measurements (M) are based on crop field trials with different levels of abiotic stress, preferably drought stress.

5. Method of any of the preceding claims, comprising the step:
determining the new metabolite features (Mn) combining the received metabolite measurements (M) based on assignments of the received metabolite measurements (M) to different ontologies (F1, F2) and/or based on a ratio between product metabolite measurements and substrate metabolite measurements (F3).

6. Method of claim 5, wherein
the ontologies (F1, F2) comprise different levels;
wherein preferably different levels are associated to different generalizations of metabolite measurements (M).

7. Method of any of the preceding claims, comprising the steps:
validating the yield loss prediction data (Yp) and providing validation data (V) by comparing the yield loss prediction data (Yp) with the actual yield data (Ya) of the respective crop plant (50); and
adjusting the model (13) based on the validation data (V).

8. Method of any of claim 7, comprising the step
adjusting a parametrization (P) of a machine learning algorithm determining the model (13) based on the validation data (V);
wherein the machine learning algorithm comprises several combined machine learning algorithms, in particular three combined machine learning algorithms.

9. Method of any of claims 7 or 8, comprising the step
determining a best new metabolite feature (Mb) from the new metabolite features (Mn) based on the validation data (V); wherein
the best metabolite feature (Mb) comprises the metabolite measurements (M) with the highest impact on the expected yield loss; and
wherein preferably the best metabolite feature (Mb) comprises metabolite measurements (M) extracted by polar gas chromatography (GCP).

10. Method of any of the preceding claims, wherein
the yield loss prediction data (Yp) is determined within the vegetative growth stage of the crop plant (50).

11. Control unit (10) being configured for executing a method of any of claims 1 to 10.

12. Yield evaluation platform (100), comprising:
a hyperspectral sensor (31) configured for determining hyperspectral data (Dh) of a crop plant (50);
a profiling platform (20) configured for determining metabolite measurements (M) from a crop plant sample (S); and
a control unit (10) of claim 11.

13. Plant breeding method, comprising the steps:
cultivating crop plants (50);
determining yield loss prediction data (Yp) of the respective crop plant (50) using the method of any of claims 1 to 10; and
selecting the crop plants (50) with a predicted yield loss (Yp) below a predetermined threshold for future breeding cycles.

14. Farming method, comprising the steps:
determining yield loss prediction data (Yp) of the respective crop plant (50) using the method of any of claims 1 to 10;
providing farmers with an expected yield loss of the crop plant (50) depending on the determined yield loss prediction data (Yp); and
adjusting the farming conditions by the farmer depending on the expected yield loss of the crop plant (50).

15. Use of new metabolite features (Mn) combining received metabolite measurements (M) of a crop plant sample (S) for yield loss prediction of the crop plant (50).
